(19) Europäisches Patentamt
European Patent Office
Office européen des brevets

(11) Numéro de publication : **0 165 919**
**B1**

# (12) FASCICULE DE BREVET EUROPÉEN

(45) Date de publication du fascicule du brevet : **09.08.89**

(51) Int. Cl.$^4$ : **C 07 D207/27**, A 61 K 31/40

(21) Numéro de dépôt : **85870068.5**

(22) Date de dépôt : **14.05.85**

## (54) (R)-alpha-éthyl-2-oxo-1-pyrrolidineacétamide.

(30) Priorité : **15.05.84 GB 8412358**

(43) Date de publication de la demande :
**27.12.85 Bulletin 85/52**

(45) Mention de la délivrance du brevet :
**09.08.89 Bulletin 89/32**

(84) Etats contractants désignés :
**AT BE CH DE FR GB IT LI LU NL SE**

(56) Documents cités :
**FR-A- 2 081 508**
**FR-A- 2 368 275**

(73) Titulaire : **U C B, S.A.**
**326, Avenue Louise**
**B-1050 Bruxelles (BE)**

(72) Inventeur : **Gobert, Jean**
**120, rue du Cornet**
**B-1040 Bruxelles (BE)**
Inventeur : **Giurgea, Corneliu**
**5, avenue de la Jonction**
**B-1060 Bruxelles (BE)**
Inventeur : **Geerts, Jean-Pierre**
**12, Habaru**
**B-6737 Leglise (BE)**
Inventeur : **Bodson, Guy**
**248, rue de la Chevratte**
**B-6739 Bellefontaine Tintigny (BE)**

(74) Mandataire : **Dusseldorp, Raymond et al**
**U.C.B. S.A. Département D.T.B. 326, avenue Louise,**
**Bte 7**
**B-1050 Bruxelles (BE)**

**EP 0 165 919 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue St-Denis, 75001 Paris, France

## Description

La présente invention se rapporte à un composé nouveau, le (R)-alpha-éthyl-2-oxo-1-pyrrolidineacétamide ainsi qu'à des procédés pour le préparer. Elle concerne également les compositions thérapeutiques renfermant ledit composé.

Dans le brevet britannique n° 1 309 692 au nom de la demanderesse, on décrit l'alpha-éthyl-2-oxo-1-pyrrolidineacétamide (point de fusion 122 °C) et on indique que les substances de ce type sont utilisables dans le domaine thérapeutique, par exemple pour le traitement du mal de mouvement, des hyperkinésies, des hypertonies et de l'épilepsie.

D'autre part, on y mentionne aussi que ces composés peuvent trouver une application dans le domaine des troubles de la mémoire dans des conditions normales ou pathologiques.

Poursuivant ses travaux de recherche dans ce domaine, la demanderesse a préparé et isolé l'énantiomère dextrogyre de l'alpha-éthyl-2-oxo-1-pyrrolidineacétamide et elle a trouvé que ce composé se distingue de manière tout à fait imprévue de la forme racémique connue

(1) par une activité mnésique environ 10 fois plus élevée et

(2) par une toxicité 3 fois plus faible.

De cet ensemble imprévisible de propriétés, il résulte que l'énantiomère dextrogyre de l'alpha-éthyl-2-oxo-1-pyrrolidineacétamide convient beaucoup mieux pour le traitement des insuffisances cérébrales, des troubles de la mémoire, des difficultés tant de concentration mentale que d'apprentissage et d'études.

C'est pourquoi la présente invention a pour objet l'énantiomère dextrogyre de l'alpha-éthyl-2-oxo-1-pyrrolidineacétamide qui possède la configuration absolue R, ledit composé étant substantiellement exempt de l'énantiomère lévogyre ayant la configuration absolue S.

Le (R)-alpha-éthyl-2-oxo-1-pyrrolidineacétamide conforme à la présente invention ne peut pas être obtenu directement à partir de la forme racémique par séparation des deux énantiomères. Il peut être préparé par l'un ou l'autre des procédés suivants :

(a) on fait réagir l'acide (R)-alpha-éthyl-2-oxo-1-pyrrolidineacétique successivement avec (1) un halogénoformiate d'alkyle de formule HalCOOZ dans laquelle Hal représente un atome d'halogène et Z un radical alkyle ayant 1 à 4 atomes de carbone et (2) de l'ammoniac. L'halogénoformiate d'alkyle est de préférence du chloroformiate d'éthyle.

Cette réaction est généralement effectuée dans le dichlorométhane, à une température comprise entre — 10 et — 60 °C.

L'acide (R)-alpha-éthyl-2-oxo-1-pyrrolidineacétique, utilisé dans cette réaction, peut être obtenu à partir de l'acide (±)-alpha-éthyl-2-oxo-1-pyrrolidineacétique racémique, par résolution chimique selon les méthodes connues en soi, par exemple en formant un sel de cet acide avec une base optiquement active et en isolant le sel formé avec l'acide (R)-alpha-éthyl-2-oxo-1-pyrrolineacétique, par cristallisations successives dans un solvant approprié (p. ex. le benzène).

A titre d'exemples de bases optiquement actives pouvant être utilisées pour cette résolution, on peut citer des alcaloïdes comme la brucine, la quinine, la strychnine, la quinidine, la cinchonidine et des amines comme l'alpha-méthyl-benzylamine et la déhydroabiétylamine (cf. S.H. WILEN et al., Tetrahedron, 33, (1977), 2725-2736). Des résultats particulièrement favorables sont obtenus en utilisant l'alpha-méthyl-benzylamine et la déhydroabiétylamine.

Quant à l'acide (±)-alpha-éthyl-2-oxo-1-pyrrolidineacétique racémique utilisé comme matière première, il peut être obtenu par saponification des esters alkylés correspondants, dont la synthèse a été décrite dans le brevet britannique n° 1 309 692.

(b) on cyclise un (R)-2-amino-butanamide de formule

$$X—CH_2CH_2—Y—NHCH(C_2H_5)CONH_2 \qquad (A)$$

dans laquelle

X représente un radical ZOOC— ou $HalCH_2$—, Z étant un radical alkyle ayant 1 à 4 atomes de carbone et Hal un atome d'halogène, de préférence le chlore ou le brome, et

Y représente un radical —$CH_2$— ou —CO—,

avec la restriction que lorsque X représente un radical ZOOC—, Y est un radical —$CH_2$— et lorsque X représente un radical $HalCH_2$—, Y est un radical —CO—.

La cyclisation du (R)-2-amino-butamide de formule A est effectuée dans un solvant inerte, tel que le toluène ou le dichlorométhane, à une température comprise entre O °C et la température d'ébullition du solvant. Cette cyclisation s'opère en présence d'une substance basique, en tant que catalyseur. Ce catalyseur est la 2-hydroxypyridine, lorsque le composé de formule A est un ester (X = ZOOC—) et le bromure de tétrabutylammonium lorsque le composé de formule A est un halogénure (X = $HalCH_2$—).

Lorsque X représente un radical ZOOC— et Y est un radical —$CH_2$—, le composé de formule A est un (R)-4-[[1-(aminocarbonyl)propyl]amino]-butyrate d'alkyle de formule ZOOC-

$CH_2CH_2CH_2NHCH(C_2H_5)CONH_2$, dans laquelle Z a la signification donnée plus haut. Ce dernier peut être préparé par condensation de (R)-2-amino-butanamide avec un 4-halogénobutyrate d'alkyle de formule $ZOOCCH_2CH_2CH_2$—Hal dans laquelle Z a la signification donnée plus haut et Hal est un atome d'halogène.

Lorsque X représente un radical $HalCH_2$— et que Y est alors un radical —CO—, le composé de formule A est un (R)-N-[1-(aminocarbonyl)propyl]-4-halogéno-butanamide de formule Hal-$CH_2CH_2CH_2CONHCH(C_2H_5)CONH_2$, dans laquelle Hal a la signification donnée plus haut. Ce dernier peut être préparé par condensation de (R)-2-amino-butanamide avec un halogénure de 4-halogénobutyryle de formule $HalCH_2CH_2CH_2COHal$ dans laquelle Hal est un atome d'halogène.

La réaction entre le (R)-2-amino-butanamide d'une part et le 4-halogénobutyrate d'alkyle ou l'halogénure de 4-halogéno-butyryle d'autre part, est généralement effectuée dans un solvant inerte, tel que le benzène, le toluène, le dichlorométhane ou l'acétone, à une température comprise entre — 5 et + 100 °C et en présence d'un accepteur d'acide tel qu'une base organique tertiaire (p. ex. la triéthylamine) ou une base minérale (p. ex. le carbonate ou l'hydroxyde de potassium ou de sodium).

Lorsque X représente un radical $HalCH_2$— et Y un radical —CO—, il n'est pas indispensable d'isoler le composé de formule A obtenu à partir des matières premières citées plus haut. En effet, le composé de formule A, obtenu in situ, peut être cyclisé directement en le (R)-alpha-éthyl-2-oxo-1-pyrrolidineacétamide conforme à la présente invention (voir l'exemple 4 ci-après).

Le (R)-2-amino-butanamide utilisé comme matière première, peut être obtenu à patir de l'acide (R)-2-amino-butyrique par ammonolyse de l'ester méthylique correspondant selon la méthode décrite par K. FOLKERS et al. dans J. Med. 14, (6), (1971), 484-7.

Les exemples qui suivent illustrent l'invention sans la limiter. Dans ces exemples, la pureté optique des produits obtenus a été vérifiée par la détermination calorimétrique desenthalpies différentielles (C. FOUQUEY et J. JACQUES, Tetrahedron, 23, (1967), 4009-19).

## Exemple 1

a) Préparation du sel de (S)-alpha-méthyl-benzylamine de l'acide (R)-alpha-éthyl-2-oxo-1-pyrrolidineacétique

Dans un ballon de 4 litres, on met en suspension 513 g (3 moles) d'acide (±)-alpha-éthyl-2-oxo-1-pyrrolidineacétique racémique dans 1,26 litre de benzène anhydre. A cette suspension, on ajoute une solution contenant 181,5 g (1,5 mole) de (S)-(-)-alpha-méthyl-benzylamine et 151,8 g (1,5 mole) de triéthylamine dans 2 litres de benzène anhydre. On porte ensuite le mélange à reflux jusqu'à dissolution complète. On refroidit et on laisse cristalliser pendant quelques heures. On filtre les cristaux et on les lave par deux fois avec 400 ml de benzène. On obtient ainsi 337 g de sel (S)-alpha-méthyl-benzylamine de l'acide (R)-alpha-éthyl-2-oxo-1-pyrrolidineacétique.

P.F. : 145-149 °C. Rendement : 76,9 %.

Ce sel peut être purifié par chauffage à reflux pendant 4 heures dans 3 litres de benzène. On refroidit et on filtre. On recueille ainsi 297,7 g du sel souhaité.

P.F. : 149-152 °C. Rendement : 68 %.

b) Préparation de l'acide (R)-alpha-éthyl-2-oxo-1-pyrrolidine-acétique

On dissout 297,7 g du sel obtenu en a) ci-dessus dans O,6 litre d'eau. On y ajoute lentement 147,3 g d'une solution d'hydroxyde de sodium à 30 %. Le pH atteint 12,6 et la température ne dépasse pas 25 °C. On agite la solution encore pendant 20 minutes et on extrait l'alpha-méthyl-benzylamine libérée par 7 fois 150 ml de benzène.

On acidifie ensuite la phase aqueuse jusqu'à une valeur de pH de 1,1 par addition de 188 ml chlorhydrique 6 N.

On agite le mélange pendant 45 minutes et on extrait l'acide libéré par 5 fois 200 ml de dichlorométhane. On réunit les phases organiques, on les sèche sur sulfate de magnésium et on évapore le solvant. On obtient 170,5 g d'acide (R)-alpha-éthyl-2-oxo-1-pyrrolidineacétique.

P.F. : 126 °C. $[alpha]_D^{20}$ : + 27,3° (c = 1, acétone). Rendement : 98 %.

c) Préparation du (R)-alpha-éthyl-2-oxo-1-pyrrolidine-acétamide

On met en suspension 17,1 g (0,1 mole) d'acide (R)-alpha-éthyl-2-oxo-1-pyrrolidineacétique dans 100 ml de dichlorométhane, refroidi à — 13 °C. On y ajoute goutte à goutte 13,9 ml de triéthylamine. A la solution obtenue, on ajoute 9,56 ml de chloroformiate d'éthyle sans dépasser — 13 °C. On agite pendant une demi-heure, puis on fait passer un courant d'ammoniac pendant environ deux heures et demie.

On laisse revenir à la température ambiante et on élimine les sels d'ammonium formés par filtration et lavage avec du dichlorométhane. On distille le solvant et on recristallise le résidu dans de l'acétate d'éthyle en présence de 10 g de tamis moléculaire (0,3 à 0,4 nm) en poudre.

On obtient 11,2 g de (R)-alpha-éthyl-2-oxo-1-pyrrolidineacétamide.

P.F. : 115-117 °C. $[alpha]_D^{25}$ : + 90,7° (c = 1, acétone). Rendement : 66 %.
Analyse pour $C_8H_{14}N_2O_2$ en %
calculé : C 56,45 H 8,29 N 16,46
trouvé : C 56,38 H 8,36 N 16,43

L'acide (±)-alpha-éthyl-2-oxo-1-pyrrolidineacétique racémique, utilisé dans cette synthèse, a été préparé de la manière décrite ci-dessous.

Dans un ballon de 20 litres contenant 3,65 kg (18,34 moles) de (±)-alpha-éthyl-2-oxo-1-pyrrolidineacétate d'éthyle, on ajoute en deux heures, à une température ne dépassant pas 60 °C, une solution contenant 788 g (19,7 mole) d'hydroxyde de sodium dans 4,35 litres d'eau. Lorsque cette addition est terminée, on porte la température du mélange jusqu'à 80 °C et on distille l'alcool formé jusqu'à ce que la température dans le milieu atteigne 100 °C.

On refroidit jusqu'à 0 °C et on ajoute en 2 heures et demie, 1,66 litre (19,80 moles) d'acide chlorhydrique 12 N. On filtre le précipité, on le lave avec deux litres de toluène et on le recristallise dans de l'alcool isospropylique. On obtient ainsi 2,447 kg d'acide (±)-alpha-éthyl-2-oxo-1-pyrrolidineacétique racémique, fondant à 155-156 °C.

Rendement : 78 %.
Analyse pour $C_8H_{13}NO_3$ en %
calculé : C 56,12 H 7,65 N 8,18
trouvé : C 55,82 H 8,10 N 7,97

## Exemple 2

a) Préparation du (R)-4-[[1-(aminocarbonyl)propyl]amino]-butyrate d'éthyle

A une suspension de 47,75 g (0,345 mole) de chlorhydrate de (R)-2-amino-butanamide ($[alpha]_D^{25}$ : — 26,1° ; c = 1, méthanol) dans 400 ml de toluène, on ajoute 143,6 ml (1,035 mole) de triéthylamine. On porte le mélange à 80 °C et on y introduit goutte à goutte 67,2 g (0,345 mole) de 4-bromo-butyrate d'éthyle.

On maintient le milieu réactionnel à 80 °C pendant 10 heures, puis on filtre à chaud pour éliminer les sels de triéthylamine. On évapore le filtrat sous pression réduite et on obtient 59 g d'un résidu huileux constitué essentiellement de produit de monoalkylation et contenant un peut de dérivé dialkylé.

Le produit, obtenu à l'état brut, a été utilisé tel quel, sans autre purification, dans la préparation du (R)-alpha-éthyl-2-oxo-1-pyrrolidine-acétamide par cyclisation.

b) Préparation du (R)-alpha-éthyl-2-oxo-1-pyrrolidine-acétamide

On met 54 g du produit brut obtenu en a) ci-dessus, en solution dans 125 ml de toluène, en présence de 2 g de 2-hydroxypyridine. On maintient le mélange à 100 °C pendant 12 heures.

On filtre à chaud un petit peu d'insoluble, puis on évapore le filtrat sous pression réduite.

On purifie le résidu par chromatographie sur une colonne de 1,1 kg de silice (diamètre de la colonne : 5 cm ; éluant : un mélange d'acétate d'éthyle, de méthanol et d'ammoniaque concentré dans des proportions en volume de 85 : 12 : 3).

Le produit isolé est recristallisé dans 50 ml d'acétate d'éthyle. On recueille ainsi 17,5 g de (R)-alpha-éthyl-2-oxo-1-pyrrolidineacétamide.

P.F. : 117 °C. $[alpha]_D^{25}$ : + 90,0° (c = 1, acétone). Rendement : 41,2 %.

## Exemple 3

a) Préparation du (R)-N-[1-(aminocarbonyl)propyl]-4-chloro-butanamide

On mélange 31,1 g (0,225 mole) de carbonate de potassium broyé et 12,47 g (0,09 mole) de chlorhydrate de (R)-2-amino-butanamide dans 160 ml d'acétone. On refroidit le mélange réactionnel à 0 °C et on y introduit goutte à goutte, une solution de 15,23 g (0,108 mole) de chlorure de 4-chlorobutyryle dans 25 ml d'acétone. Après l'addition, on laisse le mélange réactionnel revenir à la température ambiante ; on élimine l'insoluble par filtration et on évapore le filtrat sous pression réduite. Le résidu brut obtenu est agité pendant 15 minutes dans 100 ml d'éther anhydre à une température comprise entre 5 et 10 °C. On filtre le précipité on le lave avec 2 fois 30 ml d'éther et on le sèche sous vide. On obtient ainsi 16 g de (R)-N-[1-(aminocarbonyl)propyl]-4-chloro-butanamide.

P.F. : 127-129 °C. $[alpha]_D^{25}$ : + 22,2 °C (c = 1, méthanol). Rendement : 86 %.

b) Préparation du (R)-alpha-éthyl-2-oxo-1-pyrrolidine-acétamide

A 0 °C et sous atmosphère d'azote, on mélange dans 42 ml de dichlorométhane, 6,2 g (0,03 mole) de (R)-N-[1-(aminocarbonyl)propyl]-4-chloro-butanamide et 0,484 g (0,0015 mole) de bromure de tétrabutylammonium. Sans dépasser + 2 °C dans le mélange réactionnel, on ajoute en 30 minutes 2,02g (0,036 mole) d'hydroxyde de potassium pulvérulent. Le mélange est ensuite agité pendant 15 minutes, puis on laisse revenir à température ambiante. On élimine l'insoluble par filtration et on concentre le filtrat sous pression réduite. Le résidu obtenu est agité pendant 30 minutes dans 25 ml de tétrachlorure de carbone, puis filtré et séché. Le produit est recristallisé dans 45 ml d'acétate d'éthyle en présence de 1,7 g de tamis moléculaire 0,4 nm, qui est éliminé par filtration à chaud. On obtient ainsi 3,85 g de (R)-alpha-éthyl-2-oxo-1-pyrrolidineacétamide.

P.F. : 116-118 °C. $[alpha]_D^{25}$ : + 89,8° (c = 1, acétone). Rendement : 75,4 %.

## Exemple 4

Préparation du (R)-alpha-éthyl-2-oxo-1-pyrrolidineacétamide

Cet exemple illustre une variante du procédé de l'exemple 3, dans laquelle le 4-chloro-butanamide intermédiaire obtenu in situ, n'est pas isolé. A une suspension de 13,86 g (0,1 mole) de chlorhydrate de (R)-2-aminobutanamide dans 60 ml de dichlorométhane, on ajoute à la température ambiante, sous atmosphère d'azote et avec une bonne agitation, 23 g d'hydroxyde de potassium pulvérulent et 9 g d'Hyflo-cel, (Hyflo-cel est une marque commerciale).

On agite le mélange pendant 1 heure, puis on abaisse la température vers 5 °C. On ajoute alors 6,52 g (0,02 mole) de bromure de tétrabutylammonium et ensuite, en trois heures, une solution de 12,46 ml de chlorure de 4-chloro-butyryle dans 25 ml de dichlorométhane. On agite encore à 5 °C pendant une heure, puis on laisse revenir à la température ambiante et on maintient l'agitation pendant 23 heures.

On filtre le milieu réactionnel et on évapore la phase organique sous pression réduite.

On reprend le résidu à chaud par du toluène (400 % en volume par poids) et on filtre. On dissout le solide à chaud dans de l'acétate d'éthyle (400 % en volume par poids), auquel on ajoute du tamis moléculaire 0,4 nm en poudre (32 % en poids par poids). On porte ce mélange à reflux et on filtre à chaud. Après refroidissement du filtrat, le produit souhaité cristallise. On recueille ainsi 9,18 g de (R)-alpha-éthyl-2-oxo-1-pyrrolidineacétamide.

P.F. : 117 °C. $[alpha]_D^{25}$ : + 89,7° (c = 1, acétone). Rendement : 54 %.

Essais pharmacologiques

L'alpha-éthyl-2-oxo-1-pyrrolidineacétamide racémique (produit A) et le (R)-alpha-éthyl-2-oxo-1-pyrrolidineacétamide (produit B) de la présente invention ont été soumis à des tests pharmacologiques.

I. Activité/mnésique

L'activité sur la mémoire est démontrée par le test de l'antagonisme de l'effet amnésiant de l'électrochoc (S.J. SARA et M. DAVID, Psychopharmacologia, 36, (1974), 59-66).

Le principe du test est le suivant :

On observe la réaction de retrait de la patte du rat soumise à une pression croissante. La pression pour laquelle la réaction se produit est appelée « seuil de réaction ». Ce dernier est exprimé par le nombre de graduations lues directement sur l'échelle de l'appareil (analgesy-meter UGO BASILE Milan) et correspond donc à la pression minimale qui, appliquée sur la patte des animaux, provoque le retrait (séance d'apprentissage). On effectue trois mesures à un intervalle de 30 minutes.

Testés 24 heures après la fin de l'apprentissage, des animaux non traités montrent une rétention naturelle de l'épreuve antérieure qui correspond à un seuil de l'ordre de 8 à 11 graduations. L'effet amnésiant est obtenu en appliquant un électrochoc transtemporel supramaximal (100 mA, 120 Volts, 0,2 sec.) aux rats, 15 minutes après la fin de l'apprentissage. Cet effet amnésiant dû à l'électrochoc se traduit, lors de la mesure de la rétention après 24 heures, par un relèvement du seuil d'échappement qui correspond alors à celui d'animaux naïfs (sans apprentissage), c'est-à-dire un seuil compris entre 14 et 19 graduations.

On détermine pour chaque produit à tester la dose minimale active (en mg/kg) qui rétablit chez les animaux soumis à l'électrochoc, 24 heures après l'apprentissage, un seuil normal compris entre 8 et 11 graduations.

Les produits à tester sont administrés, en solution ou suspension à 10 %, par voie sous-cutanée à des groupes de 10 rats (rats Wistar femelles de 150 g) 5 minutes après la fin de l'apprentissage. En même temps, un groupe témoin de 10 rats ne reçoit qu'une solution aqueuse de chlorure de sodium à 0,9 %.

Dans ce test, l'énantiomère dextrogyre de l'invention (produit B) s'est révélé 10 fois plus actif que le racémate (produit A) pour préserver les animaux de l'effet amnésiant de l'électrochoc.

| Produit testé | Dose active (mg/kg) |
|---|---|
| A | 1,70 |
| B | 0,17 |

II. Toxicité

Dans le tableau suivant, on donne, pour les produits A et B, les DL 50 par voie intraveineuse et en mg/kg, déterminées chez la souris mâle et le rat mâle :

| Produit testé | DL 50 en mg/kg | |
|---|---|---|
| | souris | rat |
| A | 1790 | 1500 |
| B | 5603 | 5000 |

Il ressort de ce tableau que l'énantiomère dextrogyre de l'invention (produit B) est trois fois moins toxique que le racémate (produit A).

Le composé de la présente invention est administrable soit par voie orale sous forme de compositions solides ou liquides, par exemple sous forme de comprimés, gélules, dragées, capsules en gélatine, solutions ou sirops, soit par voie parentérale sous forme de solutions ou suspensions injectables.

Les formes pharmaceutiques telles que solutions ou comprimés sont préparées selon les méthodes couramment utilisées par les pharmaciens. On mélange le composé de l'invention avec un véhicule solide ou liquide, non toxique, pharmaceutiquement acceptable et éventuellement avec un agent dispersant, un agent stabilisant et le cas échéant, avec des agents colorants, édulcorants, etc.

Les excipients pharmaceutiques solides pour la préparation de comprimés ou de capsules sont, par exemple, l'amidon, le talc, le carbonate de calcium, le lactose, le sucrose, le stéarate de magnésium, etc.

Le pourcentage de produit actif dans les compositions pharmaceutiques peut varier dans des limites très larges selon le mode d'administration et l'état du patient.

La posologie humaine peut varier entre 250 mg à 4 g par jour.

A titre d'exemples non limitatifs d'une composition contenant le composé de l'invention, on donne ci-après un exemple d'une solution buvable et d'une gélule n° 0 utilisable per os :

Ampouole de solution buvable

| | |
|---|---|
| produit B | 2,5 g |
| sorbitol (à 70 % dans l'eau) | 3,0 g |
| glycérine | 2,5 g |
| glycamil | 0,025 g |
| méthylparaben | 0,0135 g |
| propylparaben | 0,0015 g |
| sodium saccharinate | 0,06 g |
| essence de réglisse | 0,05 g |
| eau purifiée ad. | 10 ml |

Gélule n° 0 à 500 mg

| | |
|---|---|
| Produit B | 500 mg |
| avicel (*) | 50 mg |
| stéarate de magnésium | 5 mg |

(*) Avicel est une marque commerciale pour une cellulose microcristalline

Il a été proposé récemment d'utiliser pour le traitement chez l'homme des déficiences de la mémoire associées à la sénescence, une combinaison de thérapies consitant à administrer un médicament qui stimule le métabolisme du cerveau, par exemple le piracétam (2-oxo-1-pyrrolidine-acétamide) et un précurseur de choline, tel que la lécithine ou un sel de choline.

Selon le brevet américain n° 4 385 053, ces deux substances (piracétam et précurseur de choline) co-agissent synergiquement en procurant une amélioration significative des capacités mémorielles chez des personnes âgées atteintes de déficiences de la mémoire et en particulier de démence sénile de type Alzheimer.

La demanderesse a observé un effet de synergie similaire entre l'énantiomère dextrogyre de l'invention et un précurseur de choline. L'énantiomère dextrogyre conforme à l'invention peut donc également être utilisé avantageusement en association avec un précurseur cholinergique du système nerveux central, pour le traitement des déficiences de la mémoire associées à la sénescence, la démence sénile de type Alzheimer, etc., en vue d'obtenir un effet clinique bénéfique sur les facultés d'acquisition et de rétention de la mémoire et sur les facultés mentales en général.

Par précurseur cholinergique, on entend non seulement la choline ou un de ses sels, mais également

toute substance qui libère de la choline dans l'organisme, comme par exemple la décithine ou la phosphatidylcholine. Pour obtenir l'effet souhaité, il est essentiel que ledit précurseur soit capable d'augmenter à la fois le niveau de choline dans le sang et la disponibilité de ladite choline pour la synthèse d'acétylcholine dans le cerveau.

Lorsque l'énantiomère dextrogyre de l'invention est utilisé conjointement avec un précurseur cholinergique, il peut être administré avant ou après l'administration dudit précurseur. Les deux produits peuvent donc être administrés simultanément, isolément ou d'une manière étalée dans le temps, de préférence simultanément. Par ailleurs, l'énantiomère dextrogyre de l'invention peut être administré par une voie identique ou différente de celle utilisée pour l'administration du précurseur cholinergique. L'administration par voie orale des deux composés, sous les formes pharmaceutiques couramment utilisées (comprimés, gélules, solutions), s'est avérée particulièrement efficace. On peut également administrer l'énantiomère dextrogyre de l'invention et le précurseur cholinergique sous forme d'une seule unité de dosage.

Dans cette application particulière, l'énantiomère dextrogyre de l'invention est de préférence administré oralement à une dose de 100 mg à 4 g par jour, tandis que le précurseur cholinergique est administré de préférence oralement de manière à fournir aux patients une dose journalière de choline comprise entre 1 et 10 g. On administre par exemple de 2 à 25 g par jour de phosphatidylcholine ou de 1 à 30 g par jour de sel de choline. Chez des volontaires sains, on a pu observer, après une administration unique simultanée et par voie orale, de 1,5 g de l'énantiomère dextrogyre de l'invention et de 25 g de lécithine (18 g de phosphatidylcholine), un effet de synergie par analyse quantitative de l'électroencéphalogramme.

**Revendications** (pour les Etats contractants : BE, CH, DE, FR, GB, IT, LI, LU, NL, SE)

1. (R)-alpha-éthyl-2-oxo-1-pyrrolidineacétamide.

2. Procédé de préparation du (R)-alpha-éthyl-2-oxo-1-pyrrolidineacétamide, caractérisé en ce qu'on fait réagir, à une température comprise entre — 10 °C et — 60 °C, l'acide (R)-alpha-éthyl-2-oxo-1-pyrrolidineacétique successivement avec (1) un halogénoformiate d'alkyle de formule HalCOOZ dans laquelle Hal représente un atome d'halogène et Z un radical alkyle ayant 1 à 4 atomes de carbone, et (2) de l'ammoniac.

3. Procédé de préparation du (R)-alpha-éthyl-2-oxo-1-pyrrolidineacétamide, caractérisé en ce qu'on cyclise, dans un solvant inerte et en présence d'un catalyseur basique choisi parmi la 2-hydroxypyridine ou le bromure de tétrabutylammonium en présence d'hydroxyde de potassium, un (R)-2-amino-butanamide de formule

$$X—CH_2CH_2—Y—NHCH(C_2H_5)CONH_2 \qquad (A)$$

dans laquelle

X représente un radical ZOOC— ou $HalCH_2$—, Z étant un radical alkyle ayant 1 à 4 atomes de carbone et Hal un atome d'halogène, et

Y représente un radical —$CH_2$— ou —CO—,

avec la restriction que lorsque X représente un radical ZOOC—, Y est un radical —$CH_2$— et que lorsque X représente un radical $HalCH_2$—, Y est un radical —CO—.

4. Procédé selon la revendication 3, caractérisé en ce que le composé de formule A est un (R)-4-[[1-aminocarbonyl)propyl]amino]-butyrate d'alkyle de formule $ZOOCCH_2CH_2CH_2NHCH(C_2H_5)CONH_2$ et en ce qu'il est préparé par condensation de (R)-2-amino-butanamide avec un 4-halogénobutyrate d'alkyle de formule $ZOOCCH_2CH_2CH_2Hal$, Z ayant la signification donnée à la revendication 3 et Hal étant un atome d'halogène.

5. Procédé selon la revendication 3, caractérisé en ce que le composé de formule A est un (R)-N-[1-(aminocarbonyl)propyl]-4-halogénobutanamide de formule $HalCH_2CH_2CH_2CONHCH(C_2H_5)CONH_2$ et en ce qu'il est préparé par condensation de (R)-2-amino-butanamide avec un halogénure de 4-halogénobutyryle de formule $HalCH_2CH_2CH_2COHal$, Hal étant un atome d'halogène.

6. Compositions thérapeutiques renfermant une quantité thérapeutiquement efficace de (R)-alpha-éthyl-2-oxo-1-pyrrolidineacétamide en association avec des excipients pharmaceutiques solides ou liquides.

**Revendications** (pour l'Etat contractant AT)

1. Procédé de préparation du (R)-alpha-éthyl-2-oxo-1-pyrrolidineacétamide, caractérisé en ce que :

a) on fait réagir à une température comprise entre — 10 °C et — 60 °C l'acide (R)-alpha-éthyl-2-oxo-1-pyrrolidineacétique successivement avec (1) un halogénoformiate d'alkyle de formule HalCOOZ dans laquelle Hal représente un atome d'halogène et Z un radical alkyle ayant 1 à 4 atomes de carbone, et (2) de l'ammoniac ; ou

b) on cyclise dans un solvant inerte et en présence d'un catalyseur basique choisi parmi la 2-hydroxypyridine ou le bromure de tétrabutylammonium en présence d'hydroxyde de potassium, un (R)-2-amino-butanamide de formule

$$X—CH_2CH_2—Y—NHCH(C_2H_5)CONH_2 \quad (A)$$

dans laquelle

X représente un radical ZOOC— ou $HalCH_2$—, Z étant un radical alkyle ayant 1 à 4 atomes de carbone et Hal un atome d'halogène, et

Y représente un radical —$CH_2$— ou —CO—,

avec la restriction que lorsque X représente un radical ZOOC—, Y est un radical —$CH_2$— et que lorsque X représente un radical $HalCH_2$—, Y est un radical —CO—.

2. Procédé selon la revendication 1, caractérisé en ce que le composé de formule A est un (R)-4-[[1-(aminocarbonyl)propyl]amino]-butyrate d'alkyle de formule $ZOOCCH_2CH_2CH_2NHCH(C_2H_5)CONH_2$ et en ce qu'il est préparé par condensation de (R)-2-amino-butanamide avec un 4-halogénobutyrate d'alkyle de formule $ZOOCCH_2CH_2CH_2Hal$, Z étant un radical alkyle ayant 1 à 4 atomes de carbone et Hal un atome d'halogène.

3. Procédé selon la revendication 1, caractérisé en ce que le composé de formule A est un (R)-N-[1-(aminocarbonyl)propyl]-4-halogénobutanamide de formule $HalCH_2CH_2CH_2CONHCH(C_2H_5)CONH_2$ et en ce qu'il est préparé par condensation de (R)-2-amino-butanamide avec un halogénure de 4-halogénobutyryle de formule $HalCH_2CH_2CH_2COHal$, Hal étant un atome d'halogène.

**Claims** (for the Contracting States : BE, CH, DE, FR, GB, IT, LI, LU, NL, SE)

1. (R)-alpha-ethyl-2-oxo-1-pyrrolidineacetamide.

2. Process for the preparation of (R)-alpha-ethyl-2-oxo-1-pyrrolidineacetamide, characterised in that (R)-alpha-ethyl-2-oxo-1-pyrrolidineacetic acid is reacted, at a temperature between — 10 °C and — 60 °C, successively with (1) an alkyl haloformate of the formula HalCOOZ in which Hal represents a halogen atom and Z an alkyl radical having 1 to 4 carbon atoms, and with (2) ammonia.

3. Process for the preparation of (R)-alpha-ethyl-2-oxo-1-pyrrolidineacetamide, characterised in that an (R)-2-amino-butanamide of the formula

$$X—CH_2CH_2—Y—NHCH(C_2H_5)CONH_2 \quad (A)$$

in which

X represents a ZOOC— or $HalCH_2$— radical, Z being an alkyl radical having 1 to 4 carbon atoms and Hal a halogen atom, and

Y represents a —$CH_2$— or —CO— radical,

with the proviso that Y is a —$CH_2$— radical when X represents a ZOOC— radical and Y is a —CO— radical when X represents a $HalCH_2$— radical, is cyclised in an inert solvent and in the presence of a basic catalyst selected from 2-hydroxypyridine or tetrabutylammonium bromide in the presence of potassium hydroxide.

4. Process according to claim 3, characterised in that the compound of formula A is an alkyl (R)-4-[[1-(aminocarbonyl)propyl]amino]-butyrate of the formula $ZOOCCH_2CH_2CH_2NHCH(C_1H_5)CONH_2$ and that it is prepared by condensing (R)-2-amino-butanamide with an alkyl 4-halobutyrate of the formula $ZOOCCH_2CH_2CH_2Hal$, Z having the meaning given in claim 3 and Hal being a halogen atom.

5. Process according to claim 3, characterised in that the compound of formula A is an (R)-N-[1-(aminocarbonyl)propyl]-4-halobutanamide of the formula $HalCH_2CH_2CH_2CONH(C_2H_5)CONH_2$ and that it is prepared by condensing (R)-2-amino-butanamide with a 4-halobutyryl halide of the formula $HalCH_2CH_2CH_2COHal$, Hal being a halogen atom.

6. Therapeutic compositions containing a therapeutically effective amount of (R)-alpha-ethyl-2-oxo-1-pyrrolidineacetamide in association with solid or liquid pharmaceutical excipients.

**Claims** (for the Contracting State AT)

1. Process for the preparation of (R)-alpha-ethyl-2-oxo-1-pyrrolidineacetamide, characterised in that :

a) (R)-alpha-ethyl-2-oxo-1-pyrrolidineacetic acid is reacted at a temperature between — 10 °C and — 60 °C, successively with (1) an alkyl haloformate of the formula HalCOOZ in which Hal represents a halogen atom and Z an alkyl radical having 1 to 4 carbon atoms, and with (2) ammonia ; or

b) an (R)-2-amino-butanamide of the formula

$$X—CH_2CH_2—Y—NHCH(C_2H_5)CONH_2 \quad (A)$$

in which

X represents a ZOOC— or $HalCH_2$— radical, Z being an alkyl radical having 1 to 4 carbon atoms and Hal a halogen atom, and

Y represents a —$CH_2$— or —CO— radical,

with the proviso that Y is a —$CH_2$— radical when X represents a ZOOC— radical and Y is a —CO— radical when X represents a $HalCH_2$— radical, is cyclised in an inert solvent and in the presence of a basic catalyst selected from 2-hydroxypyridine or tetrabutylammonium bromide in the presence of potassium hydroxide.

2. Process according to claim 1, characterised in that the compound of formula A is an alkyl (R)-4-[[1-(aminocarbonyl)propyl]amino]-butyrate of the formula $ZOOCCH_2CH_2CH_2NHCH(C_2H_5)CONH_2$ and that it is prepared by condensing (R)-2-amino-butanamide with an alkyl 4-halobutyrate of the formula $ZOOCCH_2CH_2CH_2Hal$, Z being an alkyl radical having 1 to 4 carbon atoms and Hal a halogen atom.

3. Process according to claim 1, characterised in that the compound of formula A is an (R)-N-[1-(aminocarbonyl)propyl]-4-halobutanamide of the formula $HalCH_2CH_2CH_2CONH(C_2H_5)CONH_2$ and that it is prepared by condensing (R)-2-amino-butanamide with a halobutyryl halide of the formula $Hal-CH_2CH_2CH_2COHal$,Hal being a halogen atom.

**Patentansprüche** (für die Vertragsstaaten : BE, CH, DE, FR, GB, IT, LI, LU, NL, SE)

1. (R)-Alpha-äthyl-2-oxo-1-pyrrolidinacetamid.

2. Verfahren zur Herstellung von (R)-Alpha-äthyl-2-oxo-1-pyrrolidinacetamid, dadurch gekennzeichnet, dass man (R)-Alpha-äthyl-2-oxo-1-pyrrolidinessigsäure nacheinander mit (1) einem Alkyl-halogenformiat der Formel HalCOOZ woring Hal ein Halogenatom und Z einen Alkylrest mit 1 bis 4 Kohlenstoffatomen darstellt, und (2) Ammoniak, bei einer Temperatur zwischen — 10 °C und — 60 °C, umsetzt.

3. Verfahren zur Herstellung von (R)-Alpha-äthyl-2-oxo-1-pyrrolidinacetamid, dadurch gekennzeichnet, dass man ein (R)-2-Amino-butanamid der Formel

$$X{-}CH_2CH_2{-}Y{-}NHCH(C_2H_5)CONH_2 \qquad (A)$$

worin

X den Rest ZOOC— oder $HalCH_2$— darstellt, wobei Z ein Alkylrest mit 1 bis 4 Kohlenstoffatomen und Hal ein Halogenatom ist, und

Y den Rest —$CH_2$— oder —CO— darstellt,

mit der Einschränkung, dass Y der Rest —$CH_2$— ist, wenn X den Rest ZOOC— darstellt und dass Y der Rest —CO— ist, wenn X den Rest $HalCH_2$— darstellt, in einem inerten Lösungsmittel und in Gegenwart eines basischen Katalysators ausgewählt aus 2-Hydroxypyridin oder Tetrabutylammoniumbromid in Gegenwart von Kaliumhydroxid, cyclisiert.

4. Verfahren nach Anspruch 3, dadurch gekennzeichnet, dass die Verbindung der Formel A ein Alkyl-(R)-4-[[1-(aminocarbonyl)propyl]amino]-butyrat der Formel $ZOOCCH_2CH_2CH_2NHCH(C2H_5)CONH_2$ ist und dass sie durch Kondensation von (R)-2-Amino-butanamid mit einem Alkyl-4-halogenbutyrat der Formel $ZOOCCH_2CH_2CH_2Hal$, wobei Z die in Anspruch 3 angegebene Bedeutung hat und Hal ein Halogenatom ist, hergestellt ist.

5. Verfahren nach Anspruch 3, dadurch gekennzeichnet, dass der Verbindung der Formel A ein (R)-N-[1-(aminocarbonyl)propyl]-4-halogenbutanamid ist und dass sie durch Kondensation von (R)-2-Amino-butanamid mit einem 4-Halogenbutyrylhalogenid der Formel $HalCH_2CH_2CH_2COHal$, wobei Hal ein Halogenatom ist, hergestellt ist.

6. Heilmittel, enthaltend eine therapeutisch wirksame Menge eines (R)-Alpha-äthyl-2-oxo-1-pyrrolidinacetamids in Verbindung mit festen oder flüssigen, pharmazeutischen Hilfstoffen.

**Patentansprüche** (für den Vertragsstaat AT)

1. Verfahren zur Herstellung von (R)-Alpha-äthyl-2-oxo-1-pyrrolidinacetamid, dadurch gekennzeichnet, dass man

a) (R)-Alpha-äthyl-2-oxo-1-pyrrolidinessigsäure nacheinander mit (1) einem Alkyl-halogenformiat der Formel HalCOOZ worin Hal ein Halogenatom und Z einen Alkylrest mit 1 bis 4 Kohlenstoffatomen darstellt, und (2) Ammoniak, bei einer Temperatur zwischen — 10 °C und — 60 °C, umsetzt ; oder

b) ein (R)-2-Amino-butanamid der Formel

$$X{-}CH_2CH_2{-}Y{-}NHCH(C_2H_5)CONH_2 \qquad (A)$$

worin

X den Rest ZOOC— oder $HalCH_2$— darstellt, wobei Z ein Alkylrest mit 1 bis 4 Kohlenstoffatomen und

Hal ein Halogenatom ist, und

Y den Rest —$CH_2$— oder —CO— darstellt,

mit der Einschränkung, dass Y der Rest —$CH_2$— ist, wenn X den Rest ZOOC— darstellt und dass Y der Rest —CO— ist, wenn X den Rest $HalCH_2$— darstellt, in einem inerten Lösungsmittel und in Gegenwart eines basischen Katalysators ausgewählt aus 2-hydroxypyridin oder Tetrabutylammoniumbromid in Gegenwart von Kaliumhydroxid, cyclisiert.

2. Verfahren nach Anspruch 1, dadurch gekennzeichnet, dass die Verbindung der Formel A ein Alkyl-(R)-4-[[1-(aminocarbonyl)propyl]amino]-butyrat der Formel $ZOOCCH_2CH_2CH_2NHCH(C_2H_5)CONH_2$ ist und dass sie durch Kondensation von (R)-2-Amino-butanamid mit einem Alkyl-4-halogenbutyrat der Formel $ZOOCCH_2CH_2CH_2Hal$, wobei Z die in Anspruch 1 angegebene Bedeutung hat und Hal ein Halogenatom ist, hergestellt ist.

3. Verfahren nach Anspruch 1, dadurch gekennzeichnet, dass der Verbindung der Formel A ein (R)-N-[1-(aminocarbonyl)propyl]-4-halogenbutanamid ist und dass sie durch Kondensation von (R)-2-Amino-butanamid mit einem 4-Halogenbutyrylhalogenid der Formel $HalCH_2CH_2CH_2COHal$, wobei Hal ein Halogenatom ist, hergestellt ist.